# FASCICULE DE BREVET EUROPEEN

(11) **EP 0 649 293 B1**
(45) Date de publication et mention de la délivrance du brevet: **09.04.1997**
(21) Numéro de dépôt: 93914789.8
(22) Date de dépôt: 30.06.1993
(51) Int. Cl.: A61F 2/08, A61B 17/56

(54) **PROTHESE INTERVERTEBRALE PERMETTANT UNE STABILISATION ROTATOIRE ET DE FLEXION-EXTENSION**
ZWISCHEN-WIRBEL-PROTHESE ZUR DREH-,BEUGUNGS-UND STREKKUNGS-STABILISIERUNG
INTERVERTEBRAL PROSTHESIS PROVIDING ROTATIONAL AND FLEXIONAL/EXTENSIONAL STABILIZATION

(30) Priorité: 07.07.1992 FR 9208391
(43) Date de publication de la demande: 26.04.1995
(73) Titulaire: VOYDEVILLE, Gilles, F-54000 Nancy (FR)
(72) Inventeur: VOYDEVILLE, Gilles, F-54000 Nancy (FR)
(74) Mandataire: Martin, Jean-Paul
(86) Numéro de dépôt international: FR9300664
(87) Numéro de publication internationale: WO9401057

(56) Documents cités:
- EP-A- 0 249 346
- EP-A- 0 277 280
- EP-A- 0 322 334
- EP-A- 0 392 124
- FR-A- 2 662 600
- US-A- 4 643 178
- US-A- 4 917 700

## Description

La présente invention a pour objet une prothèse intervertébrale comme définie dans le préambule de la revendication 1. Une telle prothèse est connue du document EP-A-0 392 124.

On a réalisé jusqu'à présent divers types de prothèses de contention intervertébrale, soit rigides soit souples, dont la fonction est de supprimer l'instabilité rotatoire sagittale et frontale vertébrale lombaire. Les prothèses rigides comportent des plaques, des tiges avec crochets, et les prothèses souples sont formées de ligaments associés à des cales rigides ou souples.

Toutefois ces systèmes ligamentaires connus ne permettent pas de stabiliser de manière satisfaisante la flexion-extension et la rotation.

L'invention a donc pour but de réaliser une prothèse intervertébrale du type ligamentaire, agencée de manière à pouvoir stabiliser la flexion-extension et la rotation de manière entièrement satisfaisante.

Suivant l'invention, la prothèse intervertébrale comprend les caractéristiques de la revendication 1.

Suivant une forme de réalisation avantageuse de l'invention, les deux canaux de passage de la partie mince sont juxtaposés dans la partie centrale de la cale et communiquent entre eux par une ouverture médiane permettant un croisement de deux brins de la partie mince du ligament.

La prothèse selon l'invention permet donc d'exécuter simultanément une stabilisation des vertèbres en flexion-extension grâce au cerclage péri-épineux de la partie large du ligament, et une stabilisation en rotation grâce à un passage oblique entre les épineuses de la partie mince du ligament.

Ce dernier peut être réalisé en un matériau approprié, par exemple du polyester ou le matériau connu sous la marque commerciale "DACRON", fait d'un tressage dans différents sens.

D'autres particularités et avantages de l'invention apparaîtront au cours de la description qui va suivre, faite en référence au dessin annexé qui en illustre une forme de réalisation à titre d'exemple non limitatif.

La figure 1 est une vue en perspective d'une forme de réalisation du ligament de la prothèse intervertébrale selon l'invention.

Les figures 2 et 3 sont des vues en perspective partielle de tronçons des parties mince et large respectives du ligament de la Fig.1, montrant les différences de section.

La figure 4 est une vue en perspective d'une forme de réalisation de la cale de la prothèse selon l'invention.

La figure 5 est une vue de dessus de la cale de la Fig.4.

La figure 6 est une vue en perspective de la cale sous un angle différent de celui de la Fig.4.

La figure 7 est une vue en perspective de la prothèse intervertébrale des Fig.1 à 6 en cours de montage sur les apophyses épineuses de deux vertèbres lombaires adjacentes.

La figure 8 est une vue en perspective analogue à la Fig.7 montrant le montage de la prothèse terminé.

La figure 9 est une vue en élévation postérieure de vertèbres lombaires pourvues de la prothèse intervertébrale de la Fig.8.

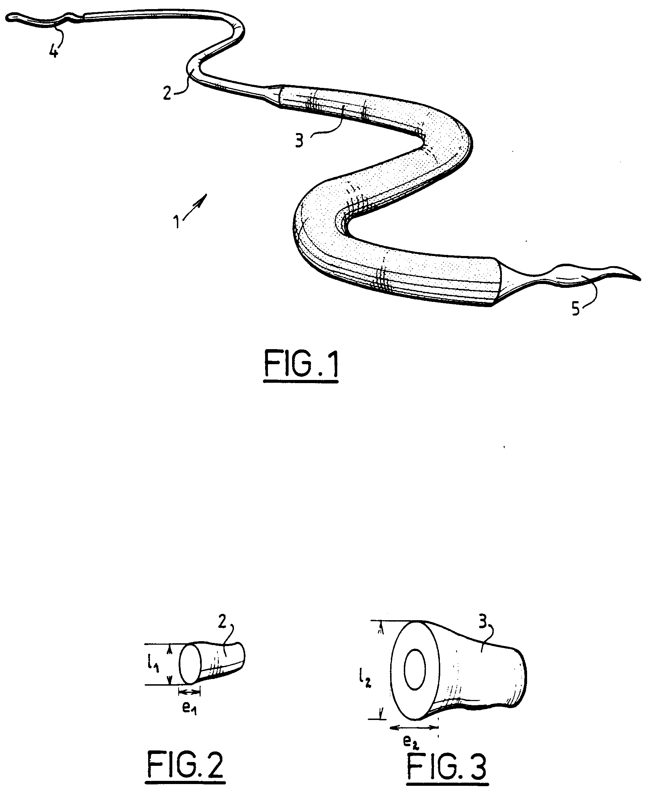

La prothèse visée par l'invention comprend un ligament souple 1 constitué de deux parties : une première partie mince 2, de faible largeur et de faible épaisseur, et une seconde partie large 3, dans le prolongement de la première partie 2, de largeur et d'épaisseur supérieures à celles de la partie 2. A titre d'exemple numérique indicatif non limitatif, la largeur l1 (Fig.2) de la partie mince 1 peut être de 4mm et son épaisseur e1 de 1mm, tandis que la largeur l2 de la partie large 3 peut être de 8mm, son épaisseur e2 étant de 2mm.

Les extrémités des deux parties 2, 3 sont prolongées par des fils 4, 5 destinés à permettre le passage du ligament 1. Le ligament est cousu sur lui-même par quatre ou cinq points en X.

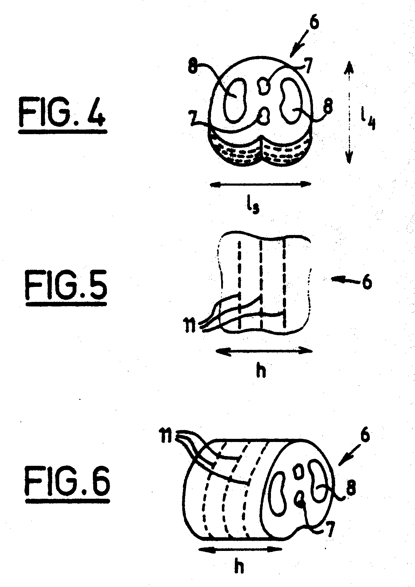

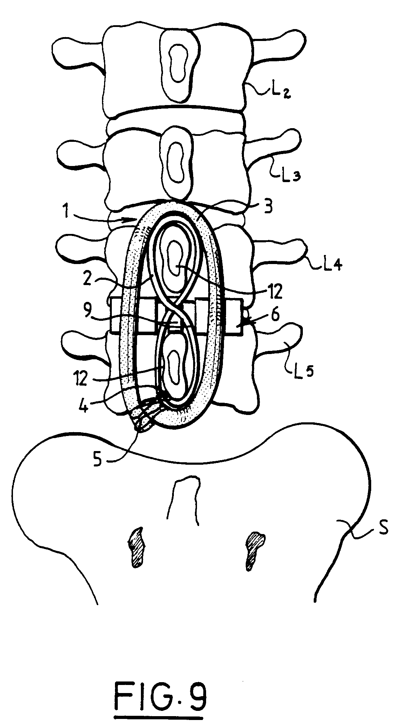

La prothèse comprend également une cale interépineuse 6 (visible notamment aux Fig.4 à 6), semi souple, dans laquelle sont percées deux paires de canaux traversants de sections différentes : une première paire de canaux 7, juxtaposés dans la partie centrale de la cale et de faible section, correspondant à la section de la partie étroite 2 du ligament 1, et deux canaux 8, placés chacun d'un côté des deux canaux 7, de section très largement supérieure à celle des canaux 7 et destinés à recevoir la partie large 3 du ligament 1. Les deux canaux centraux 7 communiquent entre eux par une ouverture médiane 9 ménagée dans la cloison de séparation des deux canaux 7 (Fig.9). Les canaux 7 et 8 sont à peu près parallèles entre eux et percent la cale 6 de part en part. La cale 6 peut avoir différentes hauteurs afin de caler les vertèbres en légère distraction.

Cette cale est réalisée dans le même matériau que le ligament 1 et rendue semi-souple au moyen par exemple de lignes de couture 11 de renforcement, qui permettent d'augmenter sa rigidité en compression.

La pose de la prothèse intervertébrale constituée du ligament 1 et d'une cale 6 s'effectue de la manière suivante.

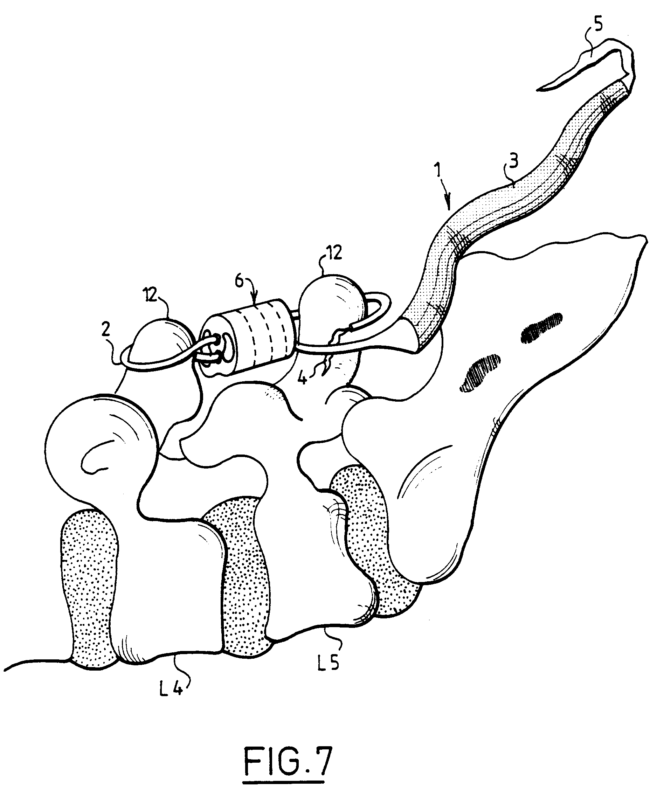

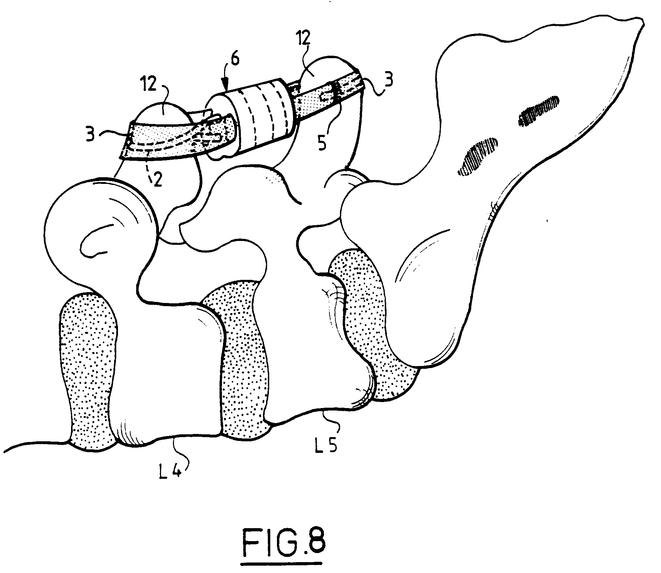

On voit aux Fig.7 à 9 les quatre dernières vertèbres lombaires L5, L4, L3, L2 et le sacrum S, la prothèse étant destinée à être posée sur les deux premières vertèbres lombaires L5, L4. Le chirurgien dispose tout d'abord la cale 6 entre les épineuses 12 de telle sorte que les canaux 7 s'étendent sensiblement de part et d'autre du plan médian des épineuses. Puis le chirurgien effectue un laçage en 8 autour des épineuses 12 au moyen de la partie mince 2 du ligament 1, en faisant passer cette partie mince successivement dans l'un des canaux 7 puis dans l'orifice central 9 et de là dans le second canal 7. Puis le chirurgien effectue un premier cerclage de l'épineuse 12 de la vertèbre L4 et réintroduit l'extrémité de la partie mince 2 dans l'un des canaux 7, l'orifice 9 et le second canal 7 en terminant le laçage par une boucle finale autour de l'épineuse 12 de la vertèbre L5. A ce stade une suture est effectuée entre les deux brins de la partie mince 2 au moyen du fil 4.

Ensuite le chirurgien effectue un cerclage interépineux au moyen de la partie large 3 autour des deux apophyses épineuses 12, en faisant passer la partie large 3 à travers les canaux latéraux 8. Une fois le bouclage complet des épineuses 12 effectué, le chirurgien termine la pose en exécutant une suture des deux extrémités de la partie large 3 l'une sur l'autre au moyen du fil 5.

A titre d'exemples numériques non limitatifs, complétant les indications données ci-dessus relativement à la section des parties étroite 2 et large 3 du ligament 1, celui-ci peut être de deux types : un pour les montages courts, un pour les montages longs. Pour les montages courts, le ligament 1 mesure par exemple 40cm, répartis entre 20cm pour la partie mince 2 et 20cm pour la partie large 3. Pour les montages longs, le ligament 1 mesure 60cm, 30cm pour la partie fine, étroite 2, 30cm pour la partie large 3. Les cales 6 correspondantes ont une largeur l3 de 15mm (Fig.4 à 6) et une longueur l4 de 15mm, tandis que leur hauteur h peut varier entre 8 et 22mm.

La résistance du ligament 1 à la traction est avantageusement supérieure à 300 kg et sa raideur est supérieure à 500 Nw/mm.

Le ligament 1 et la cale 6 sont avantageusement pourvus d'un filament radio-opaque.

La prothèse intervertébrale selon l'invention permet, grâce à sa forme et au volume restreint qu'elle occupe, de caler les vertèbres L5, L4 tant en rotation qu'en flexion-extension. En effet les ligaments utilisés jusqu'à présent ne permettent pas d'effectuer un laçage en 8 autour des vertèbres puis de faire passer à nouveau ce ligament autour de celles-ci.

Le laçage est réalisé dans tous les sens avec le ligament 1 selon l'invention, qui traverse à chaque fois la cale interépineuse 6, en rigidifiant le montage.

La cale 6 peut être considérée comme semi-élastique, car elle est suffisamment souple pour ne pas entraîner de conflit direct avec l'os, et suffisamment rigide pour maintenir une certaine distraction entre les épineuses.

En variante il est possible notamment de remplacer les fils 4, 5 de suture par tout autre moyen approprié, telle qu'une agrafe métallique ou un rivet.

Les indications pour la mise en place du ligament 1 et de la cale 6 de la prothèse visée par l'invention sont notamment les instabilités lombaires. L'instabilité lombaire est une affection relative à tous les types de rachis pathologiques, en particulier les canaux lombaires étroits, les lyses isthmiques et les hernies discales.

## Revendications

1. Prothèse intervertébrale comprenant un ligament souple (1) et une cale interépineuse (6), dans laquelle est percée une paire de canaux transversants (7; 8), adaptés pour le passage du ligament (1), caractérisée en ce que ledit
ligament souple (1) comporte une première partie mince (2) de faible largeur (l1) et de faible épaisseur (e1), et une seconde partie large (3) dans le prolongement de la première, de largeur (l2) et d'épaisseur (e2) supérieures à celles de la première partie, la première partie étant destinée à permettre un laçage en 8 autour des épineuses (12) de deux vertèbres contiguës (L5, L4), tandis que la seconde partie est adaptée pour effectuer un cerclage péri-épineux,
ladite cale interépineuse (6) est semi-souple et percée d'une deuxième paire de canaux traversants (8, 7) de section différente de celle de ladite prèmiere paire, les deux paires étant adaptées pour le passage des deux parties respectives du ligament et en ce que ladite prothèse
comprend en plus des moyens (4, 5) pour fermer le ligament sur lui même.

2. Prothèse selon la revendication 1, caractérisée en ce que les deux canaux (7) de passage de la partie mince (2) sont juxtaposés dans la partie centrale de la cale (6) et communiquent entre eux par une ouverture médiane (9) permettant un croisement de deux brins de la partie mince (2) du ligament, et les deux canaux (8) de passage de la partie large (3) sont agencés de chaque côté des orifices de passage de la partie mince.

3. Prothèse selon l'une des revendications 1 et 2, caractérisée en ce que les moyens de fermeture du ligament (1) comprennent des fils (4, 5) prolongeant les extrémités de ses deux parties, la fixation se faisant par suture en X, ou par des moyens similaires tels que rivet ou agrafe métallique.

4. Prothèse selon l'une des revendications précédentes, caractérisée en ce que la cale (6) est réalisée dans le même matériau que le ligament (1) et rendu semi-souple au moyen par exemple de coutures (11) de renforcement.

## Claims

1. Intervertebral prosthesis comprising a flexible ligament (1) and an inter-spinal wedge (6) in which are drilled a pair of through-channels (7; 8), adapted for the ligament (1) to pass through, characterised in that said flexible ligament (1) comprises a first thin part (2) of limited width (l₁) and limited thickness (e₁), and a second wide part (3) as an extension of the first part, its width (l₂) and thickness (e₂) being greater than those of the first part, the first part being intended to allow it to be laced in a figure-of-eight around the spinous processes (12) of two adjacent vertebrae (L5, L4), whilst the second part is adapted to carry out peri-spinous looping, said inter-spinal wedge (6) being semi-flexible and drilled with a second pair of through-channels (8, 7) of different cross section from said first pair, the two pairs being adapted for the two respective parts of the ligament to pass through, and in that said prosthesis further comprises means (4, 5) for closing the ligament off on itself.

2. Prosthesis according to claim 1, characterised in that the two channels (7) for the thin part (2) to pass through are juxtaposed in the central part of the wedge (6) and communicate with each other through a median opening (9) which allows two strands of the thin part (2) of the ligament to cross over, and the two channels (8) for the wide part (3) to pass through are arranged on each side of the through-openings for the thin part.

3. Prosthesis according to one of claims 1 and 2, characterised in that the means for closing off the ligament (1) comprise filaments (4, 5) which extend the ends of its two parts, fixing being achieved by an "X" suture or by similar means such as metal rivets or clips.

4. Prosthesis according to one of the preceding claims, characterised in that the wedge (6) is made of the same material as the ligament (1) and made semi-flexible by means of reinforcing seams (11), for example.

## Patentansprüche

1. Zwischen-Wirbel-Prothese mit einem weichen Band (1) und einem Zwischen-Dornfortsatz-Keil (6), in dem ein Paar durchgehender Kanäle (7; 8) für den Durchtritt des Bandes (1) ausgebildet ist, dadurch gekennzeichnet, daß das weiche Band (1) einen dünnen ersten Teil (2) mit geringer Breite (l1) und geringer Dicke (e1) und einen dicken zweiten Teil (3) in Verlängerung des ersteren aufweist, wobei die Breite (l2) und die Dicke (e2) des dicken Teils größer ist als bei dem ersten Teil, der erste Teil dazu bestimmt ist, eine 8-förmige Verschnürung um die Dornfortsätze (12) von zwei aufeinanderfolgenden Wirbeln (L5, L4) herum zubilden, während der zweite Teil dazu ausgebildet ist, die Dornfortsätze außen zu umschlingen, und der genannte Zwischen-Dornfortsatz-Keil (6) halbsteif ist und von einem zweiten Paar durchgehender Kanäle (8, 7) durchsetzt ist, deren Querschnitt von dem des ersten Paares verschieden ist, wobei die beiden Paare für den Durchtritt der beiden jeweiligen Teile des Bandes ausgebildet sind, und daß die Prothese außerdem Mittel (4, 5) zum Schließen des Bandes mit sich selbst aufweist.

2. Prothese nach Anspruch 1, dadurch gekennzeichnet, daß die beiden Kanäle (7) für den Durchtritt des dünnen Teils (2) nebeneinander im zentralen Teil des Keils (6) angeordnet sind und miteinander über eine Mittelöffnung (9) verbunden sind, die eine Überkreuzung der beiden Stränge des dünnen Teils (2) des Bandes gestattet, und die beiden Kanäle (8) für den Durchtritt des dicken Teils (3) auf jeder Seite der Durchlaßöffnungen für den dünnen Teil ausgebildet sind.

3. Prothese nach Anspruch 1 oder 2, dadurch gekennzeichnet, daß die Mittel zum Schließen des Bandes (1) Fäden (4, 5) aufweisen, die die Enden seiner beiden Teile verlängern, wobei die Befestigung durch X-Naht oder durch ähnliche Mittel wie etwa Metallnieten oder -klammern erfolgt.

4. Prothese nach einem der vorstehenden Ansprüche, dadurch gekennzeichnet, daß der Keil (6) aus dem gleichen Material wie das Band (1) hergestellt und beispielsweise durch Verstärkungsnähte (11) halbsteif gemacht ist.
